Europäisches Patentamt

European Patent Office

Office européen des brevets

(19)

(11) **EP 0 786 665 B1**

(12) **EUROPEAN PATENT SPECIFICATION**

(45) Date of publication and mention
of the grant of the patent:
**16.06.2004 Bulletin 2004/25**

(51) Int Cl.[7]: **G01N 33/58**, G01N 21/17

(21) Application number: **96101211.9**

(22) Date of filing: **29.01.1996**

(54) **Method and apparatus for immune analysis**

Verfahren und Vorrichtung zur Immunoanalyse

Procédé et dispositif pour l'analyse immunologique

(84) Designated Contracting States:
**CH DE FR GB IT LI**

(43) Date of publication of application:
**30.07.1997 Bulletin 1997/31**

(73) Proprietor: **ARKRAY, Inc.**
**Kyoto-shi, Kyoto 601 (JP)**

(72) Inventors:
• **Dou, Xiaoming,**
**c/o Kyoto Dai-ichi Kagaku Co., Ltd.**
**Minami-ku, Kyoto 601 (JP)**
• **Yamaguchi, Yoshinori,**
**Kyoto Dai-ichi Kagaku Co Ltd**
**Minami-ku, Kyoto 601 (JP)**
• **Uenoyama, Harumi,**
**c/o Kyoto Dai-ichi Kagaku Co Ltd**
**Minami-ku, Kyoto 601 (JP)**

(74) Representative: **Schoppe, Fritz, Dipl.-Ing.**
**Schoppe, Zimmermann, Stöckeler & Zinkler**
**Patentanwälte**
**Postfach 246**
**82043 Pullach bei München (DE)**

(56) References cited:
**EP-A- 0 254 430**       **EP-A- 0 654 670**
**EP-A- 0 714 024**       **FR-A- 2 691 546**
**US-A- 4 208 185**

• **DATABASE WPI Section Ch, Week 9617 Derwent**
**Publications Ltd., London, GB; Class B04, AN**
**96-163262 XP002006729 & JP-A-08 043 390**
**(KYOTO DAIICHI KAGAKU KK) , 16 February**
**1996**

EP 0 786 665 B1

## Description

BACKGROUND OF THE INVENTION

Field of the Invention

The present invention relates to a method of and apparatus for immunologically analyzing an object of measurement in the field of clinical testing, biochemical sample measurement, quality control of drugs and the like.

Description of the Background Art

Immunological analysis methods employing antigen-antibody reaction include fluorescent immunoassay and luminous immunoassay. In any one of these methods, an antigen which is labeled with a fluorescent material or a chemiluminescent material is reacted to an antibody with an antigen which is an object of measurement in competition, to form an immune complex which is a molecular complex by the antigen-antibody reaction for measuring fluorescence or luminescence from the label, thereby quantitatively analyzing the target material.

On the other hand, a method of adding an antigen or an antibody of an object of measurement to an antibody or an antigen and measuring absorption or scattering of light by an immune complex which is formed by antigen-antibody reaction thereby making quantitative analysis is known as an optical measuring method utilizing neither fluorescence nor luminescence. Quantitative analysis methods utilizing light scattering phenomenona include turbidimetry and nephelometry. The former is adapted to measure transmitted light which is attenuated by absorption and scattering, while the latter is adapted to measure scattering light intensity. The method by light scattering is adapted to measure Rayleigh scattering or Mie scattering, in response to sizes of particles as measured.

A fluorescent immunoassay apparatus belonging to fluorescent immunoassay is proposed as an immunoassay apparatus employing a total reflection cell (refer to Japanese Patent Laying-Open Gazette No. 5-203574 (1993)). In this fluorescent immunoassay, an antibody is fixed to a surface of a total reflection prism, an antigen contained in a sample is bonded to the antibody by antigen-antibody reaction, and an antibody which is labelled with a fluorescent material is further bonded to the antigen by antigen-antibody reaction. Thereafter B-F separation is performed to remove an unreacted antibody which is labelled with the fluorescent material, and then an excitation beam is introduced into the total reflection prism to excite the labelled antigen-antibody immune complex which is constrained on the surface of the total reflection prism, for measuring fluorescence generated from the fluorescent material.

The method utilizing light scattering, which is homogeneous immunoassay, is a simple method requiring neither B-F separation nor washing. However, the method utilizing Rayleigh scattering or Mie scattering has problems of low detection sensitivity and low measuring accuracy in relation to a low concentration material.

On the other hand, the fluorescent or luminescent immunoassay requires complicated chemical treatment for labelling an antigen or an antibody with a fluorescent or chemiluminescent material. Further, most thereof is heterogeneous immunoassay, which inevitably requires B-F separation for separating an immune complex (B) making antigen-antibody reaction from an antigen or antibody (F) making no antigen-antibody reaction and washing through a large number of analytical steps.

EP-A-0 254 430 discloses an immune analysis method for qualifying or further determining an antigen or an antibody being contained in a sample by measuring the light scattered back from a point of interaction between gold colloidal particles that are labelled to an antibody or an antigen and an evanescent wave.

EP-A-0 714 024 which is a document pursuant to Article 54(3) EPC discloses a method and apparatus of determining hydrogen peroxide, the apparatus having an incident optical system, a total reflection prism, a total reflection cell and a measuring optical system receiving an outgoing beam from said prism for measuring absorbance by a sample solution present in said cell.

It is the object of the present invention to provide an immune analysis method and an immunoassay apparatus, so that performing the measurement is less complicated.

This object is achieved by a method according to claim 1 and an apparatus according to claim 6.

The inventors have made deep study in order to qualitatively and quantitatively analyze an immune complex by absorption measurement of the immune complex, to discover that absorption of the immune complex can be measured in excellent sensitivity when the immune complex is brought into a state adsorbed by noble metal colloidal particles, although no absorption having sensitivity suitable for measurement can be obtained from the immune complex itself.

An immune analysis method according to the present invention includes the steps of mixing a solution containing an antibody or an antigen which is labelled with noble metal colloidal particles with a sample solution to form a sample mixed solution for reacting an antigen or an antibody contained in the sample solution with the labelled antibody or antigen thereby forming a noble metal colloidal labelled immune complex, and employing a cell having a total reflection prism at least on one surface thereof and introducing a measuring beam of a wavelength in a range from visible to infrared regions into the total reflection prism at an angle of incidence causing total reflection thereby measuring absorption of the measuring beam caused at the interface between the total reflection prism and the sample mixed solution. Thus, the antigen or the antibody which is contained in the sample is qualified or further determined.

The noble metal colloid can be prepared from a colloid of gold, silver or copper, and a proper grain size thereof is 5 to 50 nm.

According to the present invention, absorption measurement by the measuring beam can be carried out without separating the antibody or the antigen which is labelled with the noble metal colloid or an unreacted one of the antigen or the antibody contained in the sample solution, thereby implementing homogenous immunoassay analysis. Besides, high sensitive measurement can be implemented by using the noble metal colloid.

An immunoassay apparatus which is adapted to implement the aforementioned method, comprises a total reflection cell having a total reflection prism consisting of a material having a larger refractive index than the sample mixed solution on at least one of wall surfaces defining a space in which the sample mixed solution is present, an incident optical system for introducing a measuring beam of a wavelength in a range from visible to infrared regions into the total reflection prism at an angle of incidence causing total reflection, and a measuring optical system receiving an outgoing beam from the total reflection prism for measuring absorbance by the sample mixed solution. The absorption in the total reflection is measured from intensity of attenuated total reflection.

The total reflection cell can be formed by a container type cell having only one opening for causing antigen-antibody reaction in the cell for forming a noble metal colloid labelled immune complex or making antigen-antibody reaction in another container for forming a noble metal colloid labelled immune complex and thereafter storing a sample mixed solution containing the noble metal colloid labelled immune complex, or a flow cell having a solution inlet port and a solution outlet port to be fed with a sample mixed solution after formation of a noble metal colloid labelled immune complex.

A light source included in the incident optical system can be formed in accordance with the method of claim 1, for emitting a beam of a continuous wavelength, or in accordance with both the method of claim 1 and the apparatus of claim 6, by a single-wavelength light source such as a laser unit emitting a single-wavelength beam. When the light source emits a continuous wavelength beam, spectrometry can be carried out although the incident or measuring optical system must include a spectroscope. If a single-wavelength light source is employed, on the other hand, absorption measurement only at the single wavelength of the light source is carried out with no requirement for a spectroscope.

When only an antibody or an antigen which is labelled with a noble metal colloid is present in a sample mixed solution which is present in a total reflection cell, no absorption having sensitivity suitable for measurement is recognized. When an immune complex is not labelled with a noble metal colloid, no absorption having sensitivity suitable for measurement is recognized either. Absorption having sensitivity suitable for measurement is recognized at a specific wavelength only when an immune complex which is labelled with a noble metal colloid is present. The feature of the present invention resides in this point, whereby homogeneous immunoassay analysis is enabled.

Assuming that $n_2$ represents the refractive index of a sample mixed solution containing an immune complex and $n_1$ ($n_2 < n_1$) represents the refractive index of a total reflection prism, a critical angle $\theta c$ causing total reflection is expressed as follows:

$$\theta c = \sin^{-1} (n_2/n_1)$$

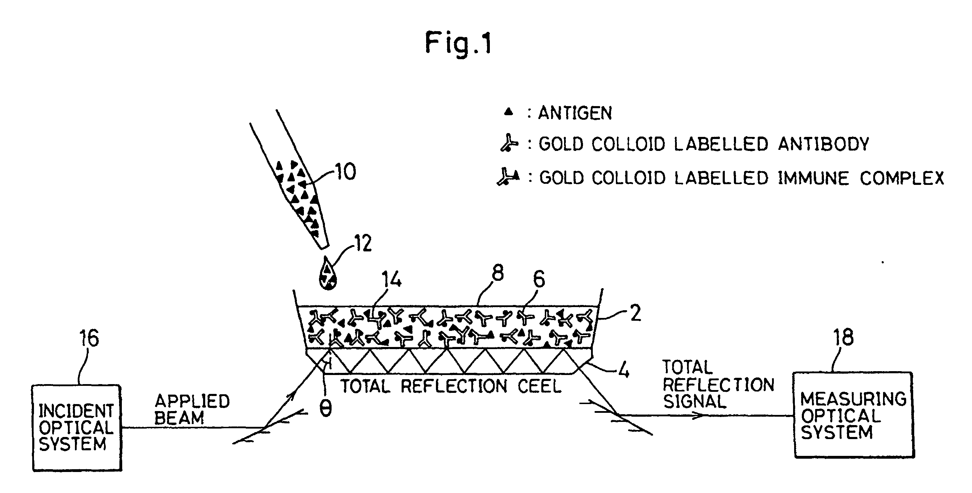

When a measuring beam into the total reflection prism from an incident optical system is introduced, an angle $\theta$ of incidence (see Fig. 1) upon the interface between the total reflection prism and the sample mixed solution is set at the following condition:

$$\theta > \theta c$$

When homogeneous immunoassay analysis is carried out, a sample mixed solution which is subjected to absorption measurement is a mixed solution containing a noble metal colloid labelled antibody (or antigen), a noble metal colloid labelled immune complex, an unreacted antigen (or antibody), biopolymer and the like.

The measuring beam is that including a continuous wavelength beam in a proper region among near infrared, mid infrared and far infrared regions, or a single-wavelength beam.

Absorption in the total reflection prism is expressed by absorbance A as follows:

$$A = N \cdot \alpha \cdot de \cdot \log e$$

where N represents the number of total reflection times in the total reflection prism, $\alpha$ represents the absorption coefficient of the sample mixed solution, and de represents an optical path length along which the measuring beam penetrates into the sample mixed solution in single total reflection.

According to the present invention, absorption by a noble metal colloid labelled immune complex which is present in a sample mixed solution is measured with a total reflection cell at high sensitivity. Absorption having sensitivity suitable for measurement is measured only in an immune complex which is in a state labelled with a noble metal colloid and no absorption having sensitivity suitable for measurement is recognized in only an antibody or an antigen which is labelled with a noble metal colloid or in an immune complex which is in a state not labelled with a noble metal colloid, whereby qualification and determination of the immune material can be carried out by absorption by total reflection measurement without performing B-F separation, while immunoassay can be carried out through a simple operation and a simple measuring apparatus.

The foregoing and other objects, features, aspects and advantages of the present invention will become more apparent from the following detailed description of the present invention when taken in conjunction with the accompanying drawings.

BRIEF DESCRIPTION OF THE DRAWINGS

Fig. 1 schematically illustrates an embodiment of the present invention;

Fig. 2 is a block diagram showing a measuring apparatus operating in accordance with a method according to claim 1;

Fig. 3 illustrates spectra of a gold colloid labelled immune complex and a gold colloid labelled antibody;

Fig. 4 illustrates spectra of the gold colloid labelled immune complex and an immune complex not labelled with a noble metal colloid;

Fig. 5 illustrates a total reflection absorption spectral difference spectrum of the gold colloid labelled immune complex based on the spectra shown in Fig. 3;

Fig. 6 illustrates concentration dependency of absorbance at 2942.5 cm$^{-1}$ with respect to antibody IgG concentration in the immunoassay method according to the present invention;

Fig. 7 illustrates concentration dependency of absorbance at 2888.1 cm$^{-1}$ with respect to antibody IgG concentration in the immunoassay method according to the present invention;

Fig. 8 illustrates concentration dependency of absorbance at 1112.4 cm$^{-1}$ with respect to antibody IgG concentration in the immunoassay method according to the present invention;

Fig. 9 illustrates concentration dependency of absorbance at 1043.3 cm$^{-1}$ with respect to antibody IgG concentration in the immunoassay method according to the present invention;

Fig. 10 illustrates concentration dependency of absorbance at 993.8 cm$^{-1}$ with respect to antibody IgG concentration in the immunoassay method according to the present invention;

Fig. 11A is a front sectional view showing an exemplary total reflection cell having only one opening, and Fig. 11B shows a front sectional view and a plan view illustrating another total reflection cell having only one opening respectively; and

Fig. 12A is a perspective view and front sectional view showing illustrates an exemplary throwaway total reflection cell, and Figs. 12B to 12E are perspective views and front sectional views showing other exemplary total reflection flow cells respectively.

DESCRIPTION OF THE PREFERRED EMBODIMENTS

Fig. 1 schematically illustrates an embodiment of the present invention. A total reflection cell 2 has a total reflection prism 4 at least on one surface thereof. A mixed solution 8 containing a gold colloid labelled antibody 6 which is adsorbed on a gold colloid serving as an exemplary noble metal colloid is stored in the total reflection cell 2, and a sample solution 12 containing an antigen 10 causing antigen-antibody reaction with the antibody 6 is added thereto, so that the antibody 6 and the antigen 10 make antigen-antibody reaction in the mixed solution 8 stored in the total reflection cell 2, thereby forming a gold colloid labelled immune complex 14. A measuring beam is introduced into the total reflection prism 4 from an incident optical system 16 at an angle θ of incidence causing total reflection, whereby the measuring beam is transmitted through the total reflection prism 4 while being totally reflected, and slightly penetrates into the mixed solution 8 through the interface between the total reflection prism 4 and the mixed solution 8, to be absorbed by the gold colloid labelled immune complex 14 in the mixed solution 8. An outgoing beam from the total reflection prism 4 is received by a measuring optical system 18 so that its absorbance is measured, whereby the antigen 10 contained in the sample solution 12 can be qualified and determined.

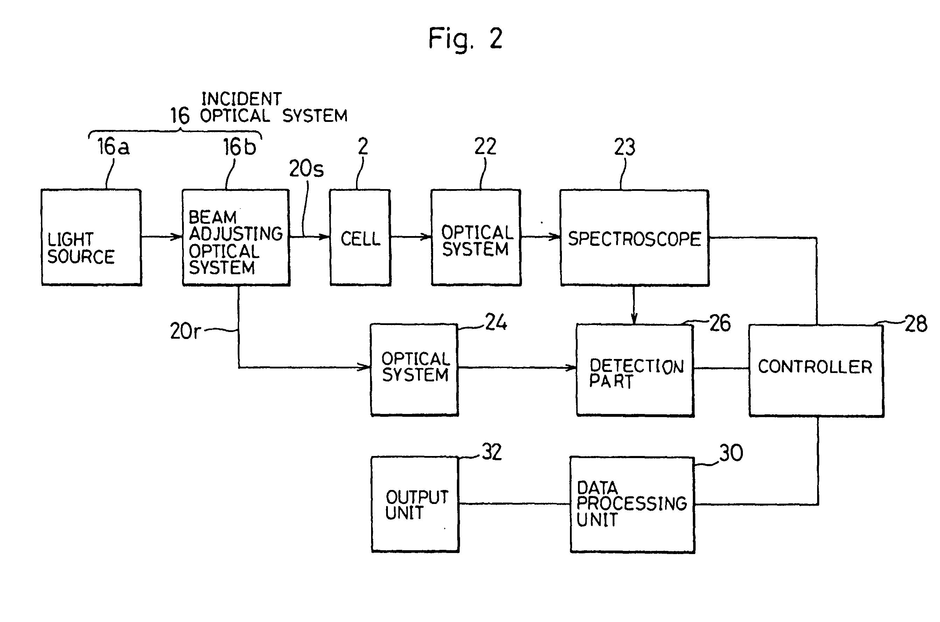

Fig. 2 schematically illustrates a measuring apparatus used to illustrate an immune analysis method according to claim 1. The incident optical system 16 includes a light source 16a emitting the measuring beam and a beam adjusting optical system 16b. The beam adjusting optical system 16b includes an optical system for converting the beam from the light source 16a to a parallel beam, a beam splitter for separating the beam into a measuring beam 20s and a reference beam 20r, and an optical system for introducing the measuring beam 20s into the total reflection prism 4 of the total reflection cell 2 at the angle of incidence causing total reflection. An optical system 22 for adjusting the luminous flux of the measuring beam 20s which is totally reflected and transmitted through the total reflection prism 4 of the total reflection cell 2 and a spectroscope 23 for receiving the measuring beam 20s adjusted by the optical system 22 and separating the received measuring beam into its spectral components are arranged on an optical path of the measuring beam 20s, so that the measuring beam 20s which is separated into its spectral components is guided to and detected by a detection part 26. The measuring optical system 18 shown in Fig. 1 includes the optical system 22, the spectroscope 23, and the detection part 26.

On the other hand, an optical system 24 for adjusting the luminous flux of the reference beam 20r is arranged on an optical path of the reference beam 20r for correcting fluctuation of the measuring beam 20s, so that the adjusted reference beam 20r is guided to and detected by the detection part 26. The detection part 26 is so formed as to correct the measuring beam 20s which is transmitted through the total reflection prism 4 and separated into its spectral components through the spectroscope 23 by intensity of the reference beam 20r indicating light source intensity thereby calculating absorbance.

Numeral 28 denotes a controller which controls the spectral operation of the spectroscope 23 for transmitting a detection output from the detection part 26 to a data processor 30. Numeral 32 denotes an output unit such as a recorder or a CRT (cathode-ray tube) outputting the result of processing in the data processor 30.

The light source 16a used to implement the immune analysis method defined in claim 1 can be formed by a fluorescent lamp, a xenon lamp, a black-body radiation source, a gas laser, a solid laser or a semiconductor laser.

The material for the total reflection prism 4 can be prepared from ZnSe, Ge, Si, $Al_2O_3$ or MgO. Only the total reflection prism 4 or overall wall surfaces of the total reflection cell 2 including the total reflection prism 4 may be made of such a material.

The spectroscope 23 can be formed by an FTIR (Fourier transformation infrared spectrophotometer), a system which is employing IR-blazed grating and IR-sensitive detector or the like. The spectroscope 23 may be included in the incident optical system 16. When a single-wavelength light source emitting a beam of a single wavelength is employed as the light source 16a, the spectroscope 23 and the controller 28 for controlling the same are unnecessary.

The cell 2 shown in Fig. 1 was employed, the spectroscope 23 of the measuring apparatus shown in Fig. 2 was formed by an FTIR, a ZnSe optical crystal was employed as the total reflection prism 4 of the cell 2, Anti-Mouse IgG (product by BioCell (U.S.A.)) absorbed on a gold colloid of 30 nm in grain size was employed as the noble metal colloid labelled antibody 6, and a mixed solution (pH 7. 65) of 0.01 M of TWEEN 21 (registered trade mark) and PBS (phosphate-buffered saline) was employed as a solvent for a mixed solution containing the antibody 6. Detection was carried out 10 times in a single measuring time of 2 minutes, and the results of the detection were estimated. The spectroscope 23 had a spectral range of 800 to 3200 cm$^{-1}$ and resolution of 2 cm$^{-1}$.

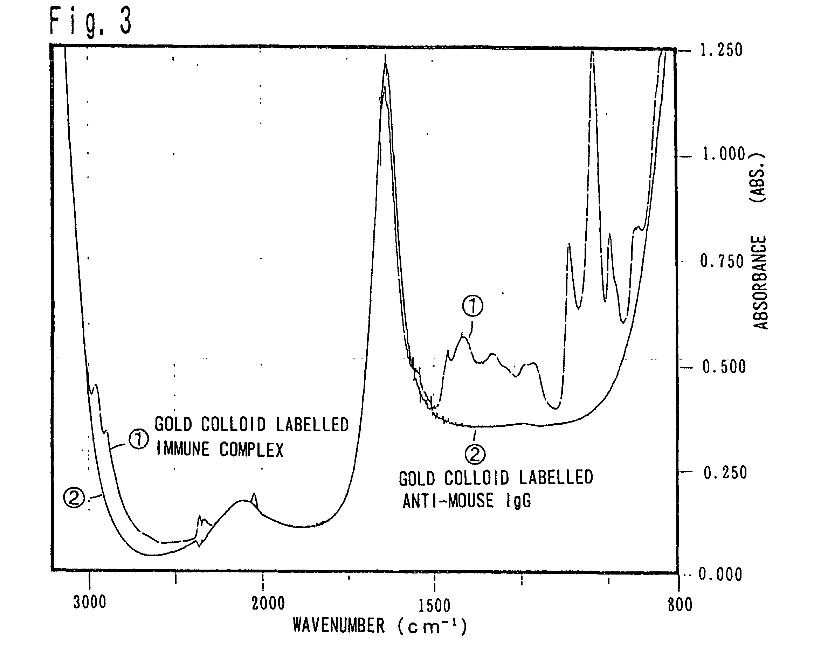

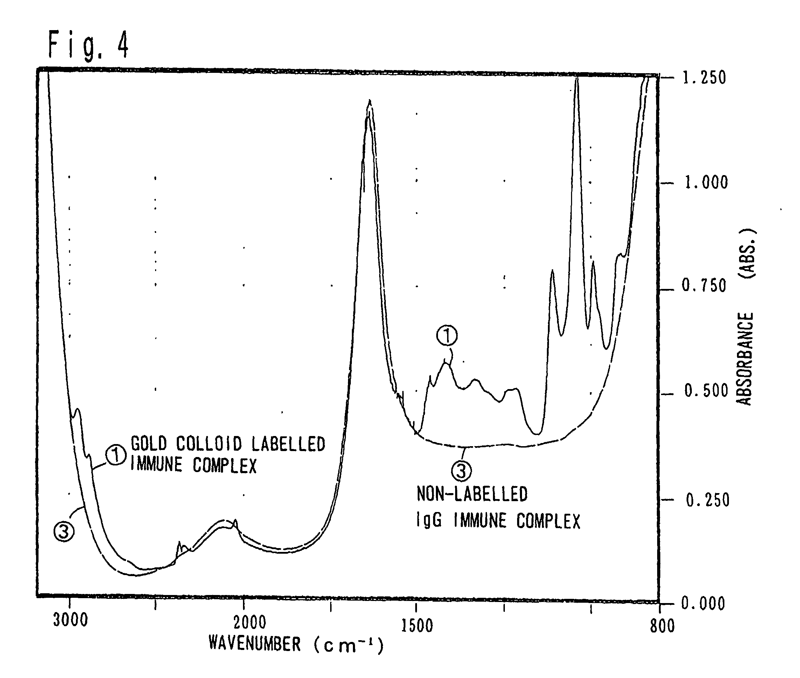

Figs. 3 and 4 show total reflection spectra of the results of the measurement. Referring to Fig. 3, a spectrum ② is that of a mixed solution containing only the aforementioned 30 nm gold colloid labelled Anti-Mouse IgG. Large absorption is that of water, while no another characteristic absorption is recognized. Referring to Fig. 4, on the other hand, a spectrum ③ is that of a mixed solution of 2 mg/ml in concentration containing only an IgG immune complex not labelled with a noble metal colloid.

No characteristic absorption is recognized in the spectrum ③ either, except that of water. On the other hand, a spectrum ① appearing in each of Figs. 3 and 4 is that of a mixed solution containing a gold colloid labelled immune complex formed by reaction of 7.5 μg/ml of the aforementioned 30 nm gold colloid labelled Anti- Mouse IgG antibody and 62. 5 μg/ml of a Mouse IgG antigen, and characteristic absorption is recognized in addition to that of water.

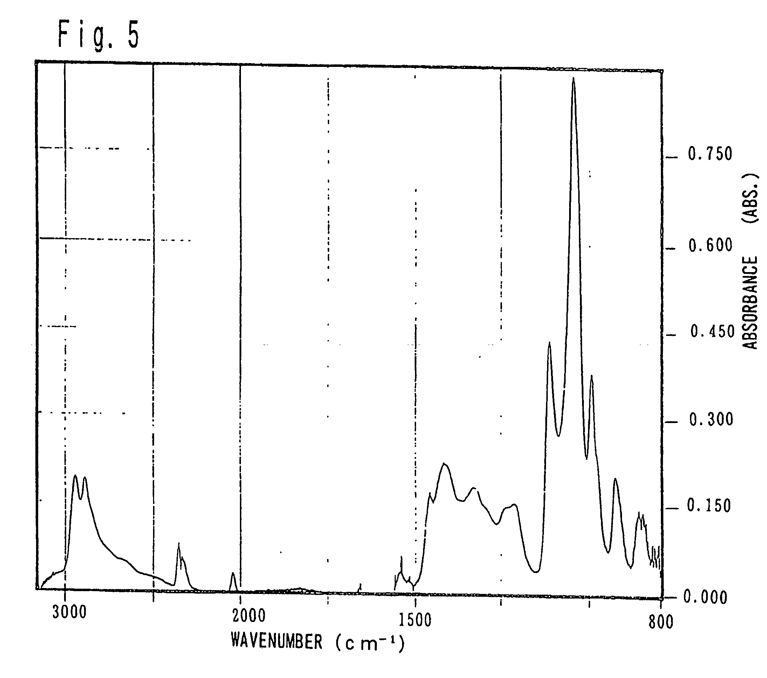

Fig. 5 illustrates a spectrum obtained by subtracting the spectrum ② from the spectrum ① in Fig. 3 as a background, in order to clearly show the absorption. This spectrum is a total reflection absorption spectral difference spectrum of the gold colloid labelled immune complex formed by reaction of 7.5 μg/ml of the 30 nm gold colloid labelled Anti-Mouse IgG antibody and 62.5 μg/ml of the Mouse IgG antigen. In this spectrum, characteristic absorption bands appear at 2888 cm$^{-1}$ and 2942 cm$^{-1}$ in the vicinity of 2900 cm$^{-1}$, and 993 cm$^{-1}$, 1043 cm$^{-1}$ and 1112 cm$^{-1}$ between the vicinity of 900 cm$^{-1}$ and 1500 cm$^{-1}$, and this is absorption by internal vibration of the IgG immune complex.

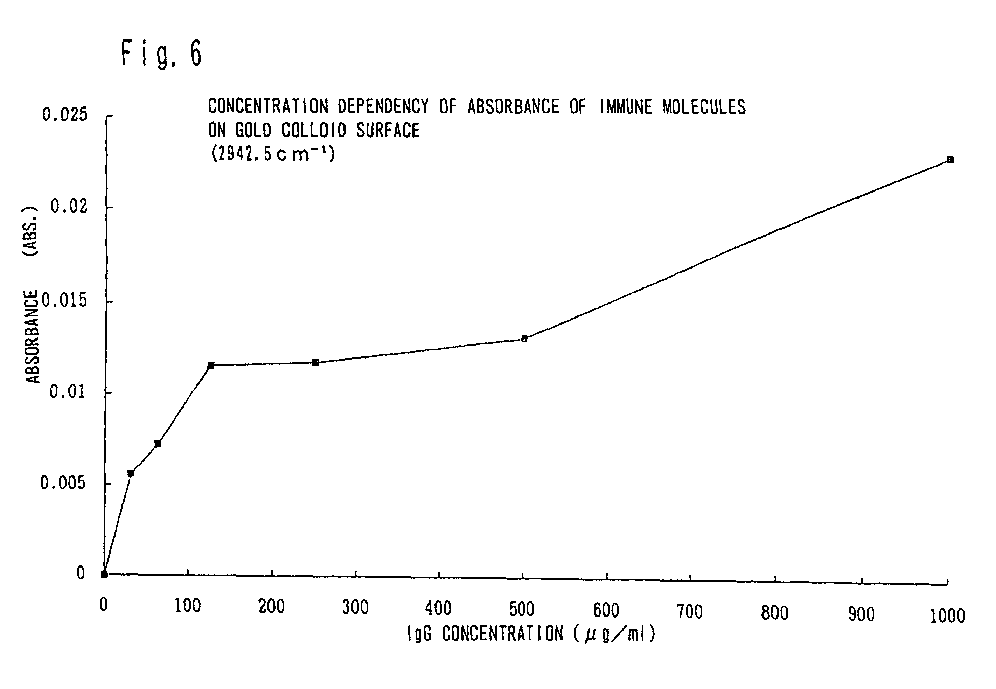

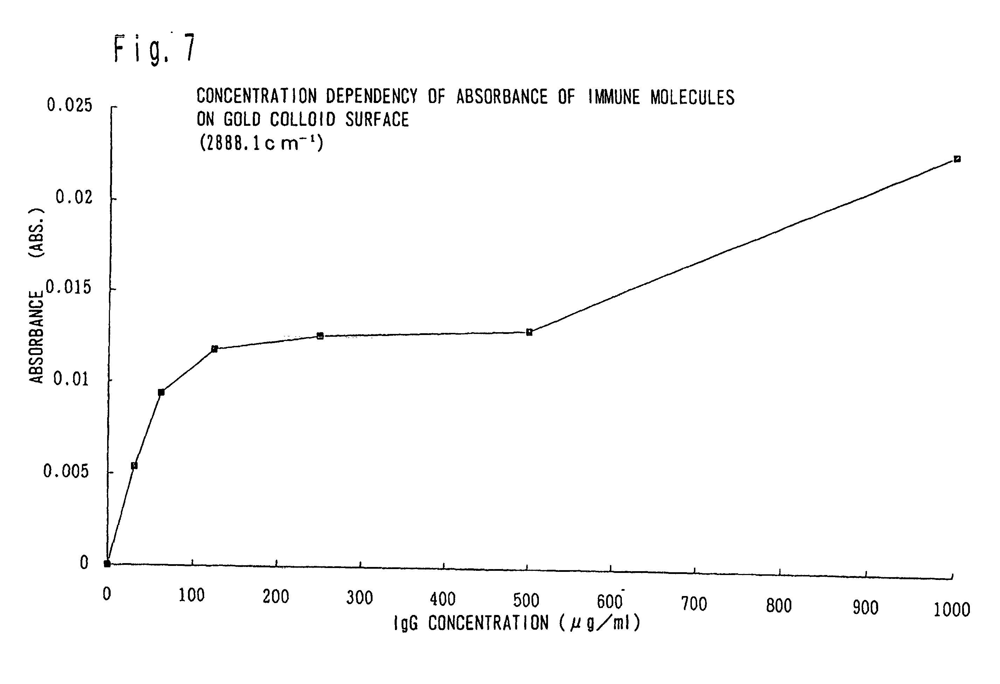

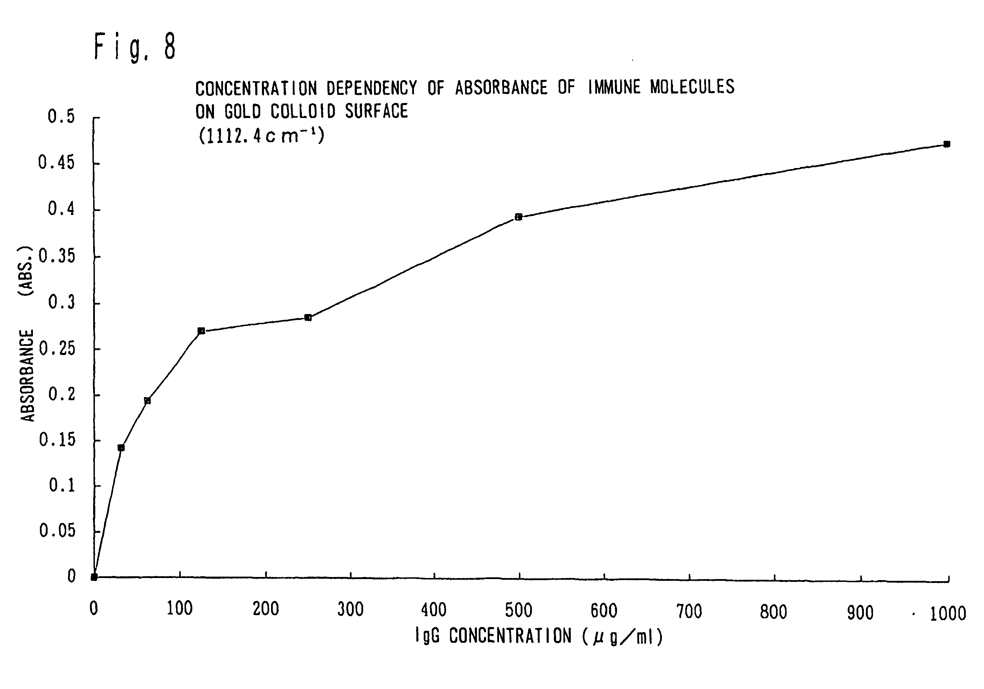

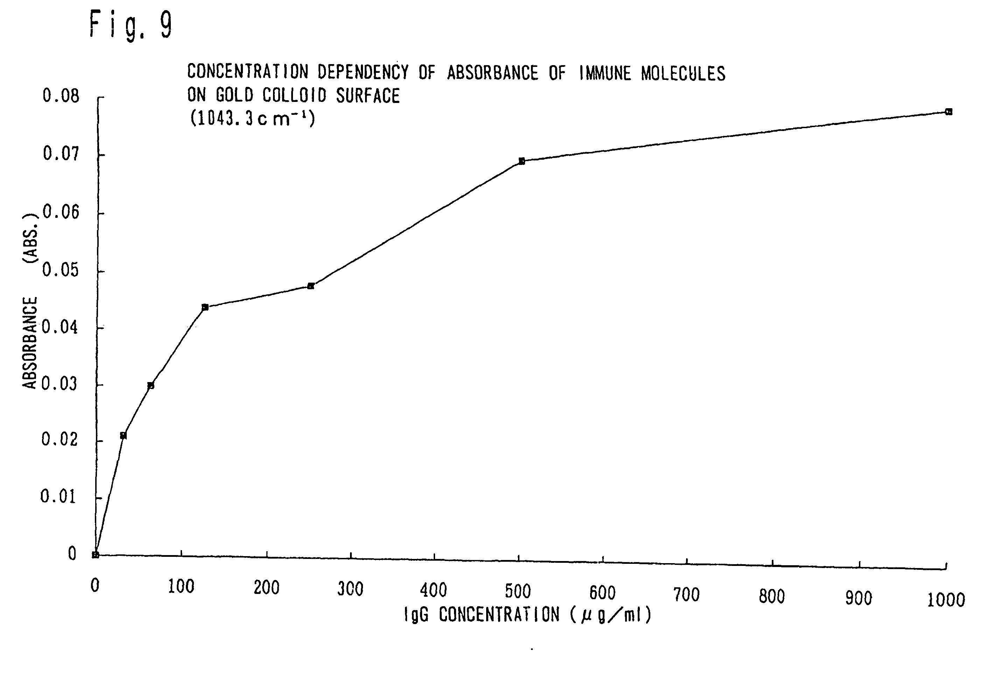

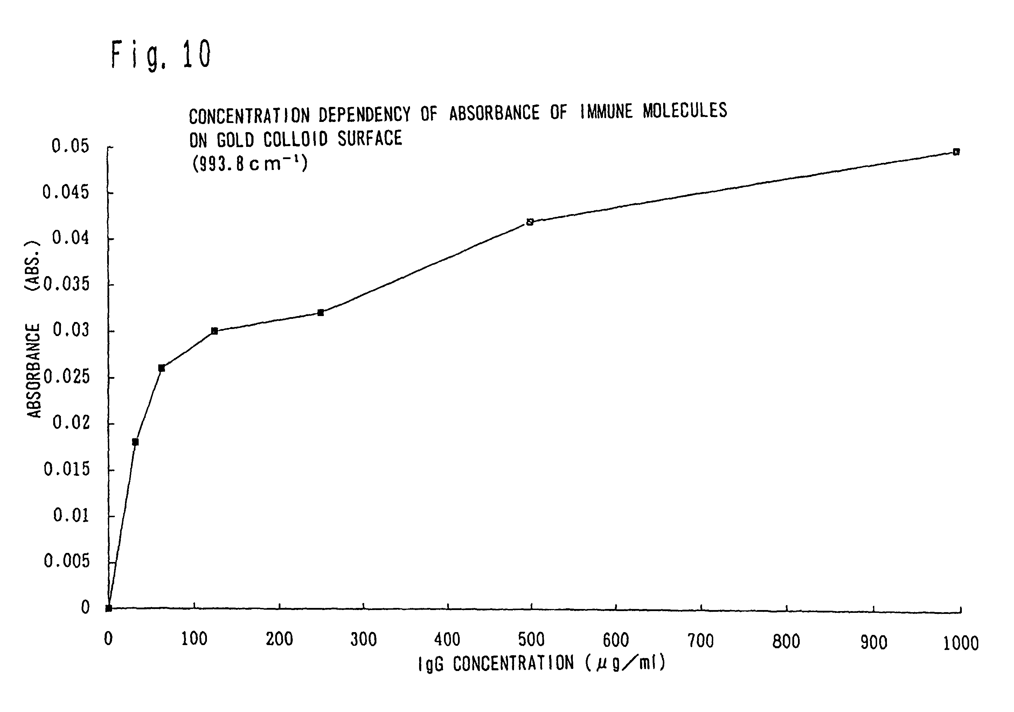

Figs. 6 to 10 illustrate concentration dependency levels of absorbance values at respective wavenumbers of 2942. 5 cm$^{-1}$, 2881. 1 cm$^{-1}$, 1112.4 cm$^{-1}$, 1043. 3 cm$^{-1}$ and 993. 8 cm$^{-1}$ as to various concentration values of Mouse IgG antigens reacted with 7.5 μg/ml of 30 nm gold colloid labelled Anti-Mouse IgG antibodies respectively. The axes of abscissas show IgG concentration values, and the axes of ordinates show absorbance values. Every one is saturated in a high concentration region of IgG, while there is a linear relation in a low concentration region, and this shows that quantitative analysis is possible by the inventive method.

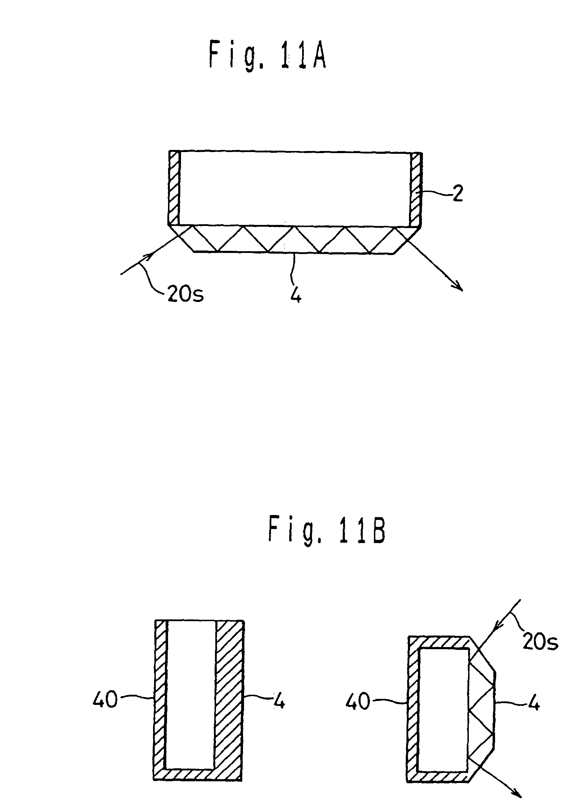

Figs. 11A and 11B show exemplary total reflection cells having only single openings. Fig. 11A illustrates the cell 2 shown in Fig. 1, having the total reflection prism 4 on its bottom surface.

Fig. 11B shows a front sectional view and a top plan view of a cell 40 having a total reflection prism 4 on its side surface.

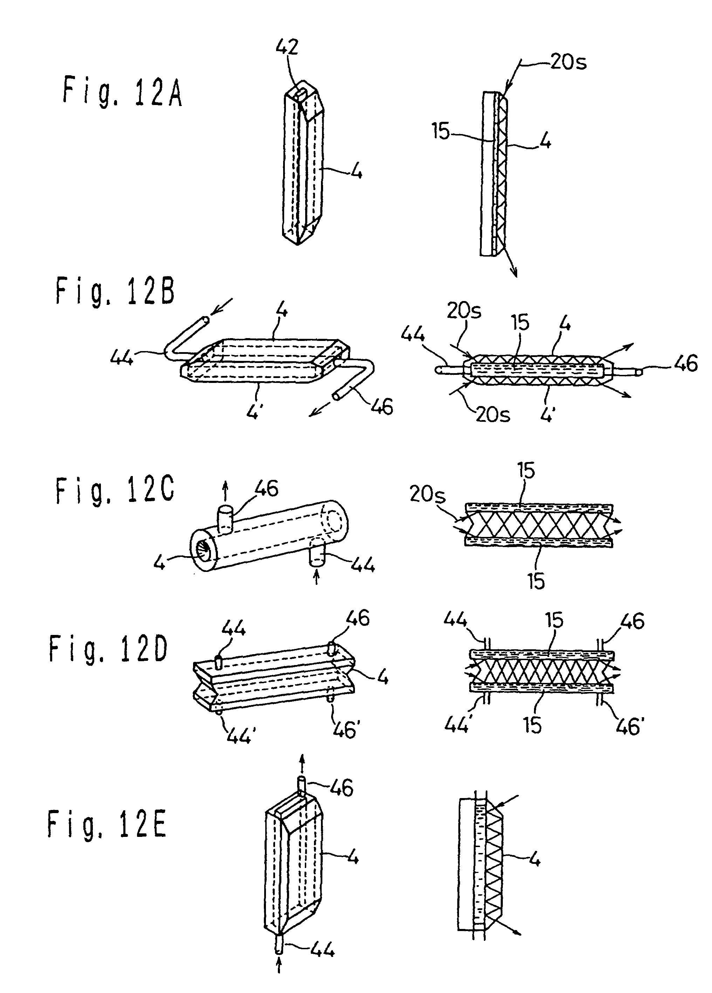

Figs. 12A to 12E show perspective views and front sectional views of cells having other shapes respectively.

Fig. 12A shows a single-sided throwaway cell, which is provided with a narrow clearance 42 for sucking a sample mixed solution by a capillary phenomenon and a total reflection prism 4 formed along the clearance 42. Numeral 15 denotes the sample mixed solution which is sucked in the clearance 42.

Fig. 12B shows an exemplary double-sided total reflection flow cell, which is provided on upper and lower surfaces thereof with total reflection prisms 4 and 4' through a space fed with a sample mixed solution. Numeral 44 denotes a sample mixed solution inlet port for the cell, and numeral 46 denotes a sample mixed solution outlet port from the cell.

Fig. 12C shows an exemplary cylindrical total reflection flow cell, which is formed to enclose a side surface of a cylindrical total reflection prism 4. A sample mixed solution 15 is fed along the cylindrical surface of the total reflection prism 4.

Fig. 12D shows another exemplary double-sided total reflection flow cell, which is so formed that a sample mixed solution 15 is fed along two opposite surfaces of a total reflection prism 4 respectively. Numeral 44, 44' denote sample mixed solution inlet ports for the cell, and numeral 46, 46' denote sample mixed solution outlet ports from the cell.

Fig. 12E shows an exemplary single-sided total reflection flow cell, having a total reflection prism 4 on one surface defining a space fed with a sample mixed solution 15.

## Claims

1.  An immune analysis method of qualifying or further determining an antigen or an antibody being contained in a sample, comprising the steps of:

    mixing a solution (8) containing an antibody or an antigen being labelled with noble metal colloidal particles with a sample solution (12) to form a sample mixed solution for reacting an antigen or an antibody being contained in said sample solution (12) with labelled said antibody or said antigen thereby forming a noble metal colloidal labelled immune complex (14);

    introducing a measuring beam of a wavelength in a range from visible to infrared regions into a total reflection prism (4) at an angle ($\theta$) of incidence causing total reflection, the reflection prism (4) being arranged on at least one surface of a total reflection cell (2) in which said sample mixed solution is present; and

    receiving a light beam being caused at the interface between said total reflection prism (4) and said sample mixed solution,

    **characterised by**
    measuring the absorption of said measuring beam being caused at the interface between said total reflection prism (4) and said sample mixed solution.

2.  The immune analysis method in accordance with claim 1, wherein

    absorption measurement by said measuring beam is carried out as homogeneous immunoassay analysis with said sample mixed solution containing both said antibody or said antigen being labelled with said noble metal colloid and said antigen or said antibody being contained in said sample solution (12) causing no antigen-antibody reaction from said labelled immune complex (14).

3.  The immune analysis method in accordance with claim 2, wherein

    said antigen-antibody reaction is caused in said total reflection cell (2), for thereafter measuring absorption of said measuring beam.

4.  The immune analysis method in accordance with claim 2, wherein

    said sample mixed solution being made to cause said antigen-antibody reaction in another container is stored in or fed to said total reflection cell (2) for measuring absorption of said measuring beam.

5.  The immune analysis method in accordance with claim 1, wherein

    said noble metal colloid is a colloid of gold, silver or copper.

6.  An immunoassay apparatus comprising:

    a total reflection cell (2) having a total reflection prism (4) on at least one of wall surfaces defining a space in which a sample mixed solution containing an immune complex (14) being labelled with a noble metal colloid is present;

    an incident optical system (16) for introducing a measuring beam of a wavelength in a range from visible to infrared regions into said total reflection prism (4) at an angle of incidence causing total reflection; and

    a measuring optical system (18) for receiving an outgoing beam from said total reflection prism (4),

    **characterised in that**
    said measuring optical system (18) is arranged to measure absorbance by said sample mixed solution, and
    said incident optical system (16) includes a single-wavelength light source for emitting a single-wavelength beam, so that absorption measurement is carried out only at said single wavelength of said light source.

7.  The immunoassay apparatus in accordance with claim 6, wherein

said total reflection cell (2) is a container type cell having only one opening.

8. The immunoassay apparatus in accordance with claim 6, wherein

said total reflection cell is a flow cell having a solution inlet port (44, 44') and a solution outlet port (46, 46'), and

the immunoassay apparatus has a means for feeding the total reflection cell with said sample mixed solution after formation of said immune complex (14) being labelled with said noble metal colloid.

**Patentansprüche**

1. Ein Immunanalyseverfahren zum Qualifizieren oder zum weiteren Bestimmen eines Antigens oder eines Antikörpers, der in einer Probe enthalten ist, das folgende Schritte aufweist:

Mischen einer Lösung (8), die einen Antikörper oder ein Antigen enthält, das mit Edelmetall-Kolloidpartikeln markiert ist, mit einer Probenlösung (12), um eine gemischte Probenlösung zu bilden, für eine Reaktion eines Antigens oder eines Antikörpers, der in der Probenlösung (12) enthalten ist, mit dem markierten Antikörper oder dem Antigen, um dadurch einen mit Edelmetallkolloid markierten Immunkomplex (14) zu bilden;

Einbringen eines Meßstrahls einer Wellenlänge in einem Bereich von sichtbaren zu Infrarot-Regionen in ein Totalreflexionsprisma (4) mit einem Einfallswinkel (θ), was eine Totalreflexion verursacht, wobei das Reflexionsprisma (4) auf zumindest einer Oberfläche einer Totalreflexionszelle (2) angeordnet ist, in der die gemischte Probenlösung vorhanden ist; und

Empfangen eines Lichtstrahls, der an der Grenzfläche zwischen dem Totalreflexionsprisma (4) und der gemischten Probenlösung verursacht wird,

**gekennzeichnet durch**
Messen der Absorption des Meßstrahls, die an der Grenzfläche zwischen dem Totalreflexionsprisma (4) und der gemischten Probenlösung verursacht wird.

2. Das Immunanalyseverfahren gemäß Anspruch 1, bei dem eine Absorptionsmessung durch den Meßstrahl als homogene Immununtersuchungsanalyse ausgeführt wird, bei der die gemischte Probenlösung sowohl den Antikörper oder das Antigen enthält, das mit dem Edelmetallkolloid markiert ist, als auch das Antigen oder den Antikörper, der in der Probenlösung (12) enthalten ist, wobei keine Antigen-Antikörper-Reaktion von dem markierten Immunkomplex (14) verursacht wird.

3. Das Immunanalyseverfahren gemäß Anspruch 2, bei dem
die Antigen-Antikörper-Reaktion in der Totalreflexionszelle (2) verursacht wird, zum nachfolgenden Messen einer Absorption des Meßstrahls.

4. Das Immunanalyseverfahren gemäß Anspruch 2, bei dem
die gemischte Probenlösung, die hergestellt ist, um die Antigen-Antikörper-Reaktion in einem anderen Behälter zu verursachen, in der Totalreflexionszelle (2) gespeichert ist oder in dieselbe gespeist wird, zum Messen einer Absorption des Meßstrahls.

5. Das Immunanalyseverfahren gemäß Anspruch 1, bei dem
das Edelmetallkolloid ein Kolloid aus Gold, Silber oder Kupfer ist.

6. Eine Immununtersuchungsvorrichtung, die folgende Merkmale aufweist:

eine Totalreflexionszelle (2), die ein Totalreflexionsprisma (4) auf zumindest einer der Wandoberflächen aufweist, die einen Raum definieren, in dem eine gemischte Probenlösung, die einen Immunkomplex (14) enthält, der mit einem Edelmetallkolloid markiert ist, vorhanden ist;

ein optisches Einfallsystem (16) zum Einbringen eines Meßstrahls einer Wellenlänge in einem Bereich von sichtbaren zu Infrarot-Regionen in das Totalreflexionsprisma (4) mit einem Einfallswinkel, der eine Totalreflexion verursacht; und

ein optisches Meßsystem (18) zum Empfangen eines aus dem Totalreflexionsprisma austretenden Strahls (4),

**dadurch gekennzeichnet, daß**
das optische Meßsystem (18) angeordnet ist, um eine Absorbanz durch die gemischte Probenlösung zu messen, und
das optische Einfallsystem (16) eine Einwellenlängen-Lichtquelle umfaßt, zum Emittieren eines Einwellenlängenstrahls, so daß eine Absorptionsmessung nur bei der einen Wellenlänge der Lichtquelle ausgeführt wird.

7.  Die Immununtersuchungsvorrichtung gemäß Anspruch 6, bei der

    die Totalreflexionszelle (2) eine Zelle vom Behältertyp ist, die nur eine Öffnung aufweist.

8.  Die Immununtersuchungsvorrichtung gemäß Anspruch 6, bei der

    die Totalreflexionszelle eine Flußzelle ist, die ein Lösungseinlaßtor (44, 44') und ein Lösungsauslaßtor (46, 46') aufweist; und

    die Immununtersuchungsvorrichtung eine Einrichtung zum Speisen der Totalreflexionszelle mit der gemischten Probenlösung aufweist, nach der Bildung des Immunkomplexes (14), der mit dem Edelmetallkolloid markiert ist.


## Revendications

1.  Procédé d'analyse immunologique pour qualifier ou, par ailleurs, déterminer un antigène ou un anticorps contenu dans un échantillon, comprenant les étapes consistant à :

    mélanger une solution (8) contenant un anticorps ou un antigène étiqueté par des particules colloïdales d'un métal noble avec une solution d'échantillon (12), pour former une solution d'échantillon mélangée pour faire réagir un antigène ou un anticorps contenu dans ladite solution d'échantillon (12), ledit anticorps ou ledit antigène étant étiqueté, formant ainsi un complexe immun étiqueté par un colloïde d'un métal noble (14) ;

    introduire un faisceau de mesure d'une longueur d'onde dans une plage des zones visibles à infrarouges dans un prisme à réflexion totale (4) suivant un angle ($\theta$) d'incidence provoquant une réflexion totale, le prisme de réflexion (4) étant disposé sur au moins une face d'une cellule à réflexion totale (2) dans laquelle se trouve ladite solution d'échantillon mélangée ; et

    recevoir un faisceau de lumière dirigé vers l'interface entre ledit prisme à réflexion totale (4) et ladite solution d'échantillon mélangée,

    **caractérisé par** :

    mesure de l'absorption dudit faisceau de mesure reçu à l'interface entre ledit prisme à réflexion totale (4) et ladite solution mélangée d'échantillon.

2.  Procédé d'analyse immunologique selon la revendication 1, dans lequel :

    la mesure de l'absorption par ledit faisceau de mesure est effectuée comme analyse d'immuno-essai homogène à l'aide de ladite solution d'échantillon mélangée contenant soit ledit anticorps soit ledit antigène étiqueté par ledit colloïde d'un métal noble et ledit antigène ou ledit anticorps contenu dans ladite solution d'échantillon (12) ne provoquant aucune réaction antigène-anticorps dudit complexe immun étiqueté (14).

3.  Procédé d'analyse immunologique selon la revendication 2, dans lequel :

    ladite réaction antigène-anticorps est provoquée dans ladite cellule à réflexion totale (2), pour mesurer ensuite l'absorption dudit faisceau de mesure.

4.  Procédé d'analyse immunologique selon la revendication 2, dans lequel :

    ladite solution d'échantillon mélangée préparée pour provoquer ladite réaction antigène-anticorps dans un autre conteneur est entreposée dans ou alimentée vers ladite cellule à réflexion totale (2), pour mesurer l'absorption dudit faisceau de mesure.

5.  Procédé d'analyse immunologique selon la revendication 1, dans lequel :

    ledit colloïde d'un métal noble est un colloïde d'or, d'argent ou de cuivre.

6.  Appareil d'immuno-essai comprenant :

    une cellule à réflexion totale (2) présentant un prisme à réflexion totale (4) sur au moins l'une des faces de paroi définissant un espace dans lequel se trouve une solution d'échantillon mélangée contenant un complexe immun (14) étiqueté par un colloïde d'un métal noble ;

    un système optique incident (16) pour introduire un faisceau de mesure d'une longueur d'onde dans une plage des zones visibles à infrarouges dans ledit prisme à réflexion totale (4) suivant un angle d'incidence provoquant une réflexion totale ; et

    un système optique de mesure (18) destiné à recevoir un faisceau de sortie dudit prisme à réflexion totale (4),

    **caractérisé par le fait que**

    ledit système optique de mesure (18) convient pour mesurer le pouvoir d'absorption par ladite solution d'échantillon mélangée, et

    ledit système optique incident (16) comporte une source de lumière à longueur d'onde unique destinée à émettre un faisceau à longueur d'onde unique, de sorte que la mesure de l'absorption n'est effectuée qu'à ladite longueur d'onde unique de ladite source de lumière.

7. Appareil d'immuno-essai selon la revendication 6, dans lequel :

   ladite cellule à réflexion totale (2) est une cellule du type conteneur ne présentant qu'une seule ouverture.

8. Appareil d'immuno-essai selon la revendication 6, dans lequel :

   ladite cellule à réflexion totale est une cellule d'écoulement présentant un orifice d'entrée de la solution (44, 44') et un orifice de sortie de la solution (46, 46'), et
   l'appareil d'immuno-essai présente un moyen d'alimentation de la cellule à réflexion totale par ladite solution d'échantillon mélangée après la formation dudit complexe immun (14) étiqueté par ledit colloïde d'un métal noble.

# Fig.1

▲ : ANTIGEN

⅄ : GOLD COLLOID LABELLED ANTIBODY

⅄▲ : GOLD COLLOID LABELLED IMMUNE COMPLEX

TOTAL REFLECTION CEEL

INCIDENT OPTICAL SYSTEM

APPLIED BEAM

θ

TOTAL REFLECTION SIGNAL

MEASURING OPTICAL SYSTEM

EP 0 786 665 B1

Fig. 2

# Fig. 3

GOLD COLLOID LABELLED
IMMUNE COMPLEX

GOLD COLLOID LABELLED
ANTI-MOUSE IgG

WAVENUMBER (cm⁻¹)

ABSORBANCE (ABS.)

EP 0 786 665 B1

Fig. 4

ABSORBANCE (ABS.)

1.250  1.000  0.750  0.500  0.250  0.000

WAVENUMBER (cm⁻¹)

800  1500  2000  3000

① GOLD COLLOID LABELLED IMMUNE COMPLEX

③ NON-LABELLED IgG IMMUNE COMPLEX

13

Fig. 5

EP 0 786 665 B1

Fig. 6

CONCENTRATION DEPENDENCY OF ABSORBANCE OF IMMUNE MOLECULES
ON GOLD COLLOID SURFACE
$(2942.5 cm^{-1})$

EP 0 786 665 B1

Fig. 7

CONCENTRATION DEPENDENCY OF ABSORBANCE OF IMMUNE MOLECULES ON GOLD COLLOID SURFACE (2888.1c m$^{-1}$)

Fig. 8

CONCENTRATION DEPENDENCY OF ABSORBANCE OF IMMUNE MOLECULES
ON GOLD COLLOID SURFACE
$(1112.4\,cm^{-1})$

ABSORBANCE (ABS.)

IgG CONCENTRATION ($\mu g/ml$)

EP 0 786 665 B1

# Fig. 9

CONCENTRATION DEPENDENCY OF ABSORBANCE OF IMMUNE MOLECULES
ON GOLD COLLOID SURFACE
$(1043.3 \, \mathrm{cm^{-1}})$

X-axis: IgG CONCENTRATION ($\mu$g/ml)

Y-axis: ABSORBANCE (ABS.)

Fig. 10

CONCENTRATION DEPENDENCY OF ABSORBANCE OF IMMUNE MOLECULES
ON GOLD COLLOID SURFACE
$(993.8\,cm^{-1})$

EP 0 786 665 B1

Fig. 11A

Fig. 11B

Fig. 12A

Fig. 12B

Fig. 12C

Fig. 12D

Fig. 12E